Europäisches Patentamt

European Patent Office

Office européen des brevets

(11) Publication number: **0 174 106**
**B1**

# EUROPEAN PATENT SPECIFICATION

(45) Date of publication of patent specification: **25.10.89**

(21) Application number: **85305627.3**

(22) Date of filing: **08.08.85**

(51) Int. Cl.⁴: **A 61 K 35/66,** A 61 K 39/118, G 01 N 33/569, G 01 N 33/571, C 12 N 1/06

(54) Detection of cell membrane protein.

(30) Priority: **23.08.84 US 643403**

(43) Date of publication of application:
**12.03.86 Bulletin 86/11**

(45) Publication of the grant of the patent:
**25.10.89 Bulletin 89/43**

(84) Designated Contracting States:
**BE DE FR GB IT NL SE**

(56) References cited:
**EP-A-0 059 624**
**EP-A-0 131 142**
**GB-A-1 036 621**
**US-A-3 928 642**
**US-A-4 497 899**

**CHEMICAL ABSTRACTS, vol. 99, no. 17, 24th October 1983, page 297, no. 136277x, Columbus, Ohio, US; H.D. CALDWELL et al.: "Immunoassay for detecting Chlamydia trachomatis major outer membrane protein", & J. CLIN. MICROBIOL. 1983, 18 (3), 539-45**

(73) Proprietor: **Becton, Dickinson and Company Mack Centre Drive P.O. Box 2224 Paramus New Jersey 07652-1149 (US)**

(72) Inventor: **Mosier, Larry 10475 Cordeil Drive St. Louis Missouri (US)**
Inventor: **Petersen, John 60 Sun Valley Creve Coeur Missouri (US)**

(74) Representative: **Ruffles, Graham Keith et al MARKS & CLERK 57-60 Lincoln's Inn Fields London WC2A 3LS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

This invention relates to the detection of the principal outer membrane protein of *Chlamydia trachomatis*.

Immunoassay is in many cases the method of choice for detecting infection by microorganisms. As an aid to specific diagnosis, the assay must be capable of identifying a particular species of microorganism with a high degree of reliability. In most cases this requires the isolation of species-specific antigens for reaction with appropriate antibodies. Typical of the type of organism yielding to such analysis is *Chlamydia trachomatis*, which is one of the two microorganism species of the genus *Chlamydiaceae* order *Chlamydiales*. The other species is *Chlamydia psittaci, Chlamydia trachomatis* in its various strains is the etiologic agent for a number of human ocular and genital diseases including trachoma inclusion conjunctivitis, lymphogranuloma venereum, "nonspecific" or nongonococcal urethritis and proctitis, *C. trachomatis* infection is pervasive throughout the general population. It has been estimated for instance that *C. trachomatis* is accountable for several million cases per year of nongonococcal urethritis.

Since *C. trachomatis* mediated disease is widespread, a reliable, simple and inexpensive test for the presence of the organism is highly desirable and of great importance in order that proper treatment can be undertaken. The only serological test in current use is the microimmunofluorescence test. This test however requires that the strains of *C. trachomatis* be used as serological test antigen. In addition the facilities for conducting this test are available in only a limited number of laboratories throughout the world. The test is very laborious, time consuming and difficult to perform. Similar considerations apply to many other microorganisms.

Recently, USA Patent No. 4,118,469 noted the preparation of an antigen of *C. trachomatis* useful in serological testing for lymphogranuloma verereum and nongonococcal urethritis. Such antigen was purified from *C. trachomatis* organisms by immunoadsorption chromatography using the monospecific antiserum as a specific ligand covalently bound in an agarose gel column. This antigen had a molecular weight of only about 160,000 daltons, and in counter-immunoelectrophoresis testing was capable of detecting antibodies from the sera of lymphogranuloma venereum patients. However, when utilized in a similar test with sera of nongonococcal urethritis patients, this antigen failed to detect antibodies. It was successful, however, in detecting antibodies in two dimensional immunoelectrophoresis testing.

In any event, however, there is still great medical interest in the isolation of species-specific antigens of *C. trachomatis*, which can be used for detecting infection, preferably by commonly practiced antigen-antibody assay methods. It therefore is an object of the present invention to provide an improved method of isolating such species-specific antigens.

The present invention relates to a diagnostic testing method for detecting cell proteins of *C. trachomatis*. The detection of a cell membrane protein is indicative of infection with that microorganism in an individual. Effective detection requires that the protein be released and/or exposed from within the infective particle of a microorganism cell. Once this is done, detection may be accomplished by a variety of assays, for example, radioimmunoassay (RIA), enzyme linked immunosorbent assay (ELISA), etc.

According to the invention there is provided a method for detecting the principal outer membrane protein of *Chlamydia trachomatis*, method comprising:

taking a test sample;

forming a sample solution by mixing the sample with a first buffer solution having a pH of from 6 to 8;

adjusting the sample solution pH to a value of from 8 to 12.5 using a solution of base;

incubating the sample for a period of from 5 minutes to 30 minutes;

adding a neutralizing second buffer having a pH of from 1 to 7 to bring the pH of the sample solution to a final value of from 7 to 8; and

assaying the sample solution to detect the presence of antigens.

In a specific embodiment of this invention, a test method for releasing the principal outer membrane protein from *C. trachomatis* includes: taking a sample such as a cervical or urethral test swab, placing the sample in a first buffer solution comprising sucrose phosphate having a pH of about 7.0, mixing the swab with the buffer solution to form a sample solution, adding to the sample solution a quantity of sodium hydroxide solution having a molarity of about 0.4 thereby raising the pH of the sample solution to between 11.0 and 11.8 raising the temperature of the sample solution to about 100°C, incubating the sample solution for a period of about 15 minutes, cooling the sample solution to a temperature of from 20°C to 30°C, reducing the pH of the cooled sample solution to between 7.2 and 7.8 using a neutralizing buffer of phosphate having a pH of about 6.1, and assaying the sample to detect the presence of antigens.

The principal outer membrane protein of *Chlamydia trachomatis* is a species-specific antigen to all serotypes of those organisms and is thus useful as a diagnostic indicator. This protein comprises about 60% of the total associated outer membrane protein of *C. trachomatis*, and has a size or subunit molecular weight of between 30,000 and 44,000 daltons, with a mean molecular weight of about 39,500 daltons. Hereinafter for ease in reference, this principal outer membrane protein group will be referred to as MP 39.5, signifying "major outer membrane protein having a mean subunit molecular weight of 39,500 daltons".

A general procedure in accordance with this invention is as follows: a test sample is first mixed with a buffer salt solution having a pH of from 6 to 8. Suitable buffers include sugar phosphate buffers, such as sucrose phosphate, and other known sugar-containing buffers. Desirably, the pH of the buffer solution is from 6.8 to 7.2.

After the sample and the buffer salt solution having been mixed thoroughly, a quantity of alkali solution is added to the sample to raise the pH to between 8 and 12 or 13. Desirably the pH is from 10 to 12, with from 11.0 to 11.8 preferred. Suitable alkali solutions include those of sodium hydroxide, potassium hydroxide, trisodium phosphate, and tri(hydroxymethyl)aminomethane, with sodium hydroxide being preferred.

Once the pH has been adjusted to the desired level, the sample is incubated for a period of from 5 to 30 minutes. The incubation can suitably take place at room temperature (say 20°C) or at elevated temperature up to 105°C. Desirably, the temperature of the sample is raised to between 90°C and 100°C, with about 100°C preferred. The use of elevated temperature during the incubation has been found to increase the efficiency by which the cell membrane protein is released and/or exposed. After the incubation period, if the sample solution has been heated, it is cooled to a temperature of from 0°C to 40°C, with about 25°C preferred. Cooling is preferably by immersion in an ice bath.

After cooling the sample, a neutralizing second buffer is added, having a pH of from 1 to 7, to bring the pH of the sample to a final value of from 7 to 8. Optimum conditions for assaying are generally obtained at these pH values, i.e., neutral to slightly alkaline. Preferably the solution has a final pH of from 7.2 to 7.8. Suitable neutralizing buffers include the various phosphate buffer solutions (PBS), with sucrose phosphate preferred. Other suitable buffer solutions include citric acid, hydrochloric acid and tri(hydroxymethyl)aminomethane · HCl. Following this pH adjustment, the sample is ready for assaying without further modification.

As is apparent from the preceding description, a variety of buffer salts, alkali solutions to raise the pH, and neutralizing buffers can be used in the method of this invention, along with a broad range of incubation times and temperatures. The actual reaction components and conditions can be selected so as to integrate the method of liberating MP 39.5, or other protein, into the particular detection assay of interest, e.g., enzyme immunoassay (EIA), radioimmunoassay (RIA), latex immunoassay, etc. In general, any type of known immunoassay technique can be used.

Monospecific antibodies against MP 39.5 antigen can be generated by suitable inoculation procedures with laboratory animals such as mice or rabbits. The animal generated antibodies may be utilized in assays for Chlamydial infection in other mammals. These assays may be conducted using well-known procedures for assaying the presence of bacterial antigen in the infected subject. Once a supply of monospecific antibodies has been secured from MP 39.5 antigen-inoculated laboratory animals, either direct or indirect assay procedures can be undertaken using specimens suspected of harboring Chlamydial infections.

In a direct assay procedure, monospecific antibody against the membrane protein may be covalently or non-covalently attached to a solid phase support system. As is customary in these techniques, the support system may be glass, plastic or the like.

The solid phase support with attached monospecific antibody against the particular membrane protein may be incubated with a specimen prepared as outlined above. Monospecific antibody against the membrane protein antigen, which previously has been radiolabeled or conjugated with enzyme by known techniques, is then equilibrated against the support system. Any antigen present in the specimen and which has been bound to the antibody on the support system will in turn bind to the radiolabeled or enzyme conjugated antibody.

If radiolabeled antibody is used, the amount of residual radioactivity in the sample then may be determined. This value is compared to specimens that have been determined to be free of the membrane protein antigen. In the event enzyme conjugated antibody is used, a substitute specific for the enzyme is added to the solid support reaction mixture and the resultant color change is recorded spectrophotometrically. This color change is compared to samples known to be free of the membrane protein antigen. In this way, the presence of the antigen in specimens can be assayed directly.

Alternatively, indirect assay procedures can be used. Specifically, the antigens can be covalently or non-covalently bound to a suitable solid phase support system. A specimen prepared as outlined above is mixed with a known quantity of radiolabeled or enzyme conjugated antibody against the membrane protein antigen, previously secured from a laboratory animal source. The specimen extract-antibody mixture may then be incubated with the solid support system and its bound antigen.

The radioactivity of the solid support system is measured, or color development in the conjugated system is measured, and compared to specimens similarly treated as standards and which do not contain the antigen of interest.

The ability of the clinical sample suspected of containing the microorganism to inhibit the binding of the radiolabeled or enzyme conjugated antibodies to the solid support reveals the presence, or absence, of the membrane protein antigen in the clinical specimen. Any demonstrated inhibition indicates infection. Other suitable assay methods and variations will be apparent to those skilled in such assay techniques.

The following example illustrates the invention.

## Example

One hundred clinical swabs were obtained and tested for the presence of *Chlamydia*. Reagent formulations were prepared as follows:, where $dH_2O$ indicates deionized water.

A. Sodium hydroxide (0.42 M) solution
    34 ml 50% (12.5 M) NaOH
    q.s. to 1.0 l

B. Neutralizing buffer
    to 900 ml $dH_2O$ add:
    $NaH_2PO_4 \cdot H_2O$—13.8 g
    adjust pH to 6.10±0.05 with NaOH
    BSA 2.0 g
    $NaN_3$ 1.0 g

C. Standard diluent
    to 800 ml $dH_2O$ add:
    6.90 g $NaH_2PO_4 \cdot H_2O$
    1.000 g BSA
    0.50 g $NaN_3$
    34.25 g Sucrose
    1.044 g $K_2HPO_4$
    0.544 g $KH_2PO_4$
    25.0 ml Heat Treated Fetal Calf Serum
    25 mg Streptomycin
    50 mg Vancomycin
    12,500 units Nystatin
    adjust pH to 7.45±0.05 using 0.42 M NaOH
    q.s. to 1.0 l
    Filter through a 0.22 µm filter

D. Conjugate buffer
    to 600 ml $dH_2O$ add:
    0.532 g $KH_2PO_4$
    2.80 g $K_2HPO_4$
    0.20 g Thimerosal
    25.0 ml Heat Treated Fetal Calf Serum
    adjust pH to 7.4±0.1
    q.s. to 1.0 l
    Filter through 0.22 µm filter

E. Well wash buffer
    to 900 ml $dH_2O$ add:
    0.532 g $KH_2PO_4$
    2.800 g $K_2HPO_4$
    1.000 g BSA
    adjust pH to 7.4±0.1
    q.s. to 1.0 l with $dH_2O$

F. Substrate buffer
    to 900 ml $dH_2O$ add:
    8.203 g Sodium Acetate Anhydrous
    adjust pH with 1M Citric Acid to 6.0±0.1
    q.s. to 1.0 l with $dH_2O$

G. Tetramethyl Benzidine (TMB) substrate
    to 900 ml Dimethyl Sulfoxide (DMSO)—S-pectragrade,
    freezing point, 18°C add:
    10.0 g 3,3′,5,5′, tetramethyl benzidine
    q.s. to 1.0 l with DMSO.
    Store at room temperature

H. 0.5 *M* Hydrogen peroxide solution
    to 500 ml stabilized 3% $H_2O_2$ add 500 ml $dH_2O$ and mix.

I. Stopping solution—2 M sulfuric acid
    to 800 ml $dH_2O$ add:
    carefully 111 ml Sulfuric Acid (concentrated).
    Cool to R.T.
    q.s. to 1.0 l with $dH_2O$

J. 2 SP Transport media
    to 900 ml $dH_2O$ add:
    Sucrose 68.5 g
    $K_2HPO_4$ 2.088 g
    $KH_2PO_4$ 1.088 g
    Fetal Calf Serum (Heat Treated) 50 ml
    Streptomycin 50 mg
    Vancomycin 100 mg
    Nystatin 25,000 units
    Adjust pH to 7.0 and q.s. to 1.0 l
    filter through sterile 0.22 µm filter.

## Sample treatment

Sample dacron swabs were cut just above cotton directly into 10×75 mm glass test tubes. Two tubes were also set up with unused swabs as blanks. 500 µl of 2SP transport medium was added to each tube and the mixtures vortexed 10—20 seconds vigorously, followed by addition of 50 µl 0.42 *M* NaOH to each tube. The mixtures were then vortexed 10—20 seconds vigorously, followed by incubation at 100°C (±2°C) for 15 minutes. The tubes were then placed in an ice bath and cooled to about 25°C, after which 500 µl of the neutralizing buffer were added. The mixtures were then vortexed 10—20 seconds vigorously. Samples were then ready for assay.

## Assay protocol—Enzyme linked immuno-sorbent assay (ELISA)

Antibody coated plates were washed 3 times with wash buffer and tapped dry. One hundred microliters of standards, controls, blanks or treated samples were added to corresponding duplicate wells, followed by mixing. The controls were sample swabs known to contain elementary bodies of Chlamydia and treated as described above. A second set of controls known to contain elementary bodies were treated with 500 µl of the neutralizing buffer and 50 µl NaOH. This set of controls was untreated. The plates were then covered with plate sealers (tin foil) and incubated overnight at ambient temperature. The contents of the wells were then discarded and the plates again washed, as above. One hundred microliters of conjugate were added to each well, followed by mixing and covering with plate sealers. The plates were then incubated at 37°C for 2 to 3 h. The contents of the well were again discarded and the plate washed, as above, followed by the addition of two hundred microliters of working TMB substrate solution. The working solution had the following composition:

10.0 ml Substrate buffer
100 µl Stock TMB substrate
30 µl H₂O
Mix solution thoroughly

The mixture was incubated 30 minutes at ambient temperature with occasional mixing. The reaction was then stopped by the addition of 50 microliters of $2M$ $H_2SO_4$. The plates were read against a substrate blank prepared by adding 200 microliters substrate and 50 microliters $2M$ $H_2SO_4$ to a strip of wells, using a 450 nanometer filter.

Calculations and interpretation of results

The absorbance of the blank swabs should be comparable to the 0.0 ng/ml standard. Absorbances of standard wells, controls and samples were each averaged. The average 0.0 ng/ml absorbance was subtracted from all averaged absorbances of standards. The average absorbance of the blank swabs was subtracted from the average absorbances of all samples and controls. The corrected average absorbance of each standard was then plotted against the concentration in ng/ml, and the recoveries determined from the plotted curves. A recovery value of ≥0.5 ng/ml was considered positive. All positive samples were repeated using both the above protocol and the confirmatory assay given below.

Confirmatory assay protocol for positive samples

Using the same Chlamydia antibody coated on the ELISA wells, solutions were prepared containing 1 mg/ml in standard buffer diluent. 10×75 mm test tubes were labeled by adding 250 µl of each digested positive sample or control to be treated. 5 µl of the antibody solution were added to all tubes, which were then mixed thoroughly by vortexing and left standing for 5 minutes. To corresponding duplicate wells were added 100 µl of the antibody treated samples. Duplicate wells from aliquots of the digested samples were also set up (not treated with Ab). Assays were then performed as described above.

Interpretation of results from confirmatory testing

A positive result was assigned to samples with MP 39.5 recoveries ≥0.5 ng/ml in the wells containing the samples not treated with antibody and a significant decrease (>70% inhibition) in recovery in the sample treated with antibody. A sample assaying as positive or questionable positive when first assayed that becomes negative in the confirmatory assay was considered negative. This may result either from recovering ≤0.5 ng/ml on the repeat test (aliquots not treated with Ab) or where the aliquots treated with antibody did not show a significant decrease in recovery.

Results

Of the 100 samples tested, 62 were negative and 38 positive. Confirmation of positive results was by inhibition assay. Elementary bodies (EB) in controls were divided into treated and untreated groups to determine the efficiency of MP 39.5 liberation. Of the treated EB the average absorbance at 450 nanometers was 0.611 while the untreated EB yielded an absorbance of 0.018 at 450 nanometers.

Claims

1. A method for detecting the principal outer membrane protein of Chlamydia trachomatis, method comprising:
taking a test sample;
forming a sample solution by mixing the sample with a first buffer solution having a pH of from 6 to 8;
adjusting the sample solution pH to a value of from 8 to 12.5 using a solution of base;
incubating the sample for a period of from 5 to minutes to 30 minutes;
adding a neutralizing second buffer having a pH of from 1 to 7 to bring the pH of the sample solution to a final value of from 7 to 8; and
assaying the sample solution to detect the presence of antigens.

2. A method according to claim 1 wherein the first buffer is a sugar-containing buffer solution, especially sucrose phosphate.

3. A method according to claim 1 or 2 wherein the base comprises sodium hydroxide, potassium hydroxide, trisodium phosphate, or tri(hydroxymethyl)aminomethane.

4. A method according to claim 1, 2 or 3, wherein the incubating step includes heating the sample solution to a temperature of from 20°C to 105°C after adjusting the sample pH, incubating the sample at that temperature, and cooling the sample solution after incubation to from 0°C to 40°C, preferably about 25°C.

5. A method according to claim 4 wherein the sample solution is heated to a temperature of from 90°C to 100°C.

6. A method according to any preceding claim wherein the neutralizing second buffer comprises a phosphate, citric acid, hydrochloric acid or tri(hydroxymethyl)aminomethane · HCl solution, especially sucrose phosphate.

7. A method according to any preceding claim, wherein the final pH of the sample solution is from 7.2 to 7.8.

8. A method according to any preceding claim, wherein after mixing the sample with a first buffer solution, the pH of the sample is adjusted to between 11.0 and 11.8.

9. A method according to claim 8, wherein the pH is adjusted with sodium hydroxide.

10. A method according to any preceding claim, wherein the assaying is by radioimmunoassay.

11. A method according to any of claims 1 to 9, wherein the assaying is by enzyme linked immunoabsorbent assay.

12. A method for detecting the principal outer membrane protein of Chlamydia trachomatis, the method comprising:
taking a sample such as a cervical or urethral test swab;

placing the sample in a buffer solution comprising sucrose phosphate having a pH of about 7.0;

mixing the sample with the buffer solution to form a sample solution;

adding to the sample solution a quantity of sodium hydroxide solution having a molarity of about 0.4 thereby to raise the pH of the sample solution to from 11.0 to 11.8;

raising the temperature of the sample solution to about 100°C;

incubating the sample solution at about 100°C for a period of about 15 minutes;

cooling the sample solution to a temperature of from 20°C to 30°C;

reducing the pH of the cooled sample solution to between 7.2 and 7.8, using a neutralizing buffer of phosphate having a pH of about 6.1;

and assaying the sample to detect the presence of antigen.

**Patentansprüche**

1. Verfahren zur Bestimmung des Hauptproteins der Außenzellmembran von Chlamydia trachomatis; dieses Verfahren umfaßt folgendes:

Probenentnahme;

Herstellung einer Probenlösung durch Mischung der Probe mit einer ersten Pufferlösung mit einem pH-Wert von 6 bis 8;

Einstellung des pH-Wertes der Probenlösung mit Hilfe einer basischen Lösung auf einen pH-Wert von 8 bis 12,5;

Inkubation der Probe für einen Zeitraum von 5 Minuten bis 30 Minuten;

Zugabe einer neutralisierenden, zweiten Pufferlösung mit einem pH-Wert von 1 bis 7, um den endgültigen pH-Wert der Probenlösung auf 7 oder 8 einzustellen; sowie

analytische Untersuchung der Probenlösung auf die Anwesenheit von Antigenen.

2. Verfahren nach Anspruch 1, bei dem der erste Puffer in einer zuckerhaltigen Pufferlösung, insbesondere Saccharosephosphat, besteht.

3. Verfahren nach Anspruch 1 oder 2, bei dem die Base aus Natriumhydroxid, Kaliumhydroxid, Natriumorthophosphat oder Tri-(hydroxymethyl)-aminomethan besteht.

4. Verfahren nach Anspruch 1, 2 oder 3, bei dem die Inkubation unter anderem ein Erhitzen der Probenlösung auf eine Temperatur von 20°C bis 105°C nach Einstellung des pH-Wertes der Probe beinhaltet, ferner die Inkubation der Probe bei dieser Temperatur und danach das Abkühlen der Probenlösung auf eine Temperatur von 0°C bis 40°C, am besten auf 25°C.

5. Verfahren nach Anspruch 4, bei dem die Probenlösung auf eine Temperatur von 90°C bis 100°C erhitzt wird.

6. Verfahren nach irgendeinem der vorstehenden Ansprüche, bei dem die neutralisierende, zweite Pufferlösung aus einem Phosphat, Zitronensäure, Salzsäure oder einer Tri-(hydroxymethyl)-aminomethan · HCl-Lösung besteht, insbesondere aus Saccharosephosphat.

7. Verfahren nach irgendeinem der vorstehenden Ansprüche, bei dem der End-pH-Wert der Probenlösung zwischen 7,2 und 7,8 liegt.

8. Verfahren nach irgendeinem der vorstehenden Ansprüche, bei dem nach dem Mischen der Probe mit einer ersten Pufferlösung der pH-Wert der Probe auf einen Wert zwischen 11,0 und 11,8 eingestellt wird.

9. Verfahren nach Anspruch 8, bei dem der pH-Wert mit Natriumhydroxid eingestellt wird.

10. Verfahren nach irgendeinem der vorstehenden Ansprüche, bei dem die analytische Bestimmung durch Radioimmunoassay erfolgt.

11. Verfahren nach einem der Ansprüche 1 bis 9, bei dem die analytische Bestimmung durch Enzym-Immunosorbentassay durchgeführt wird.

12. Verfahren zur Bestimmung des Hauptproteins der Außenzellmembran von Chlamydia trachomatis; dieses Verfahren umfaßt folgendes:

Probenentnahme beispielsweise in Form eines Gebärmutterhals- oder Harnleiterabstrichs;

Einlegen der Probe in einer Pufferlösung mit Saccharosephosphat und einem pH-Wert von ca. 7,0;

Mischung der Probe mit der Pufferlösung zur Herstellung einer Probenlösung;

Zugabe von Natronlauge (Natriumhydroxidlösung) mit einer Molkonzentration von 0,4 zu der Probenlösung, um den pH-Wert der Probenlösung auf 11,0 bis 11,8 zu erhöhen;

Erhöhung der Probenlösungstemperatur auf etwa 100°C;

Inkubation der Probenlösung bei ca. 100°C für einen Zeitraum von ca. 15 Minuten;

Abkühlen der Probenlösung auf eine Temperatur von 20°C bis 30°C;

Reduzierung des pH-Wertes der abgekühlten Probenlösung auf 7,2 bis 7,8 mit Hilfe eines neutralisierenden Phosphat-Puffers mit einem pH-Wert von ca. 6,1 sowie

analytische Untersuchung der Probe auf die Anwesenheit von Antigenen.

**Revendications**

1. Procédé de détection de la protéine principale de *Chlamydia trachomatis*, procédé comprenant:

la prise d'un échantillon d'essai,

la formation d'une solution d'échantillon par mélange de l'échantillon à une première solution tampon ayant un pH compris entre 6 et 8,

le réglage du pH de la solution échantillon à une valeur comprise entre 8 et 12,5 à l'aide d'une solution d'une base,

l'incubation de l'échantillon pendant une période comprise entre 5 et 30 minutes,

l'addition d'un second tampon de neutralisation ayant un pH compris entre 1 et 7 afin que le pH de la solution d'échantillon atteigne une valeur finale de 7 à 8, et

l'analyse de la solution échantillon afin que la présence d'antigènes soit détectée.

2. Procédé selon la revendication 1, dans lequel le premier tampon est une solution tampon contenant un sucre, notamment du phosphate de saccharose.

3. Procédé selon la revendication 1 ou 2, dans lequel le base est l'hydroxyde de sodium, l'hydroxyde de potassium, le phosphate trisodique ou le tri(hydroxyméthyl)aminométhane.

4. Procédé selon la revendication 1, 2 ou 3, dans lequel l'étape d'incubation comprend le chauffage de la solution échantillon à une température comprise entre 20 et 105°C après réglage du pH de l'échantillon, l'incubation de l'échantillon à cette température, et le refroidissement de la solution échantillon après incubation à une température comprise entre 0 et 40°C, de préférence voisine de 25°C.

5. Procédé selon la revendication 4, dans lequel la solution échantillon est chauffée à une température comprise entre 90 et 100°C.

6. Procédé selon l'une quelconque des revendications précédentes, dans lequel le second tampon de neutralisation contient un phosphate, de l'acide citrique, de l'acide chlorhydrique ou une solution de tri(hydroxyméthyl)aminométhane · HCl, notamment un phosphate de saccharose.

7. Procédé selon l'une quelconque des revendications précédentes, dans lequel le pH final de la solution échantillon est compris entre 7,2 et 7,8.

8. Procédé selon l'une quelconque des revendications précédentes, dans lequel, après mélange de l'échantillon avec une première solution tampon, le pH de l'échantillon est réglé à une valeur comprise entre 11,0 et 11,8.

9. Procédé selon la revendication 8, dans lequel le pH est réglé par de l'hydroxyde de sodium.

10. Procédé selon l'une quelconque des revendications précédentes, dans lequel l'analyse est une analyse radioimmunologique.

11. Procédé selon l'une quelconque des revendications 1 à 9, dans lequel l'analyse est réalisée par un essai sur immunoabsorbant à enzyme lié.

12. Procédé de détection de la protéine principale de la membrane externe de *Chlamydia trachomatis*, le procédé comprenant:

la prise d'un échantillon tel qu'un prélèvement cervical ou urétral,

la disposition de l'échantillon dans une solution tampon contenant un phosphate de saccharose ayant un pH d'environ 7,0,

le mélange de l'échantillon à la solution tampon afin qu'une solution échantillon soit formée,

l'addition, à la solution échantillon, d'une quantité d'une solution d'hydroxyde de sodium ayant une molarité d'environ 0,4, afin que le pH de la solution échantillon soit porté à une valeur comprise entre 11,0 et 11,8,

l'élévation de la température de la solution échantillon à 100°C environ,

l'incubation de la solution échantillon à environ 100°C pendant une période d'environ 15 minutes,

le refroidissement de la solution échantillon à une température comprise entre 20 et 30°C,

la réduction du pH de la solution échantillon refroidie à une valeur comprise entre 7,2 et 7,8 par utilisation d'un tampon de neutralisation de phosphate ayant un pH d'environ 6,1, et

l'analyse de l'échantillon afin que la présence d'un antigène soit détecté.